# EUROPEAN PATENT APPLICATION

(11) **EP 1 285 632 A1**
(43) Date of publication of application: **26.02.2003**
(21) Application number: 01500212.4
(22) Date of filing: 09.08.2001
(51) Int. Cl.: A61B 17/70

(54) **Anterior cervical plate and fixation system**

(71) Applicant: Lafitt, S.A., 46988 Paterna (Valencia) (ES)
(72) Inventor: Forriol Pico, Joaquin, 46988 Paterna (Valencia) (ES)
(74) Representative: Isern-Cuyas, Maria Luisa

(57) **Abstract**

The purpose of this Patent is an "**Anterior cervical fastening device**", the main use of which is explained in the description of this invention.

Its minimum efficient structure is composed of:
Four equal screws (7) that are inserted two-by-two on one and the other side of the anterior body of the operated vertebras, with their heads (16) partially or completely screwed.
Two equal plates (1) joining the screwed screws (7) of the same side, provided with longitudinal grooves at their ends (2) in which the heads (16) of the screws (7) are inserted.
A stiff transversal connection element (9) between the plates (1), with a longitudinal groove (13).
Two screwed rods (11) that are inserted into the central orifice of the plates (1) and their projecting ends are introduced into the longitudinal groove (13) of the transversal connection (9).
Six special nuts (5) that are fastened on to the four heads (16) of the screws (7) and on to the two ends of the rods (11).

## Description

### PURPOSE

The purpose to which the invention protected by this patent refers consists of an "Anterior cervical fastening device".

This is made up of a set of mechanical means the structure of which has been designed with an arrangement that permits, by means of fastening with screws some of its component elements, maintaining the stability of the operated vertebral bodies and thus favoring the osseous fusion of grafts inserted between them.

Its function is mainly developed until the cervical vertebral levels intended to be fused become strengthened and can transmit the stresses to which the column is subjected.

### HISTORY

Anterior cervical fastening devices made up of plates in which screws of a varying diameter are inserted are known, the fastening position of which is the anterior part of the cervical vertebral body.

These devices that, at first, provide a simple and accurate means for transmitting stresses, have however some problems, such as:
- With the osseous fusion of the inserted graft (whether in the disk space of two adjacent vertebras or in the space left after removing seriously fractured vertebras or vertebras with tumors) being the main purpose of the anterior cervical fastening systems, some characteristics of the normally used plates paradoxically hinder this goal. According to the Law of Wolff, the graft will become strengthened if the stress it supports is the normal or physiological stress. But the structure of many cervical plates is rigid, with the result that they absorb all the normal or physiological stress needed to strengthen the graft. In other words, if the space does not support stress (stress shielding) due to the action of a cervical plate, it will weaken and will not favor arthodesis or osseous fusion. On the other hand, with some pathologies (fractures or tumors), it is advisable to maintain a certain degree of stiffness in the plate. In these cases, a plate that permits absorbing a certain compression stress, although not all, would be ideal.
- The cervical vertebras are of a much smaller size that the rest of the spinal column. This means that insertion of the screws is critical, as on many occasions the small anatomical dimensional variations of each individual entail important alterations on the cervical rachis when deciding the place of inserting the screws. The known plates not only do not solve this problem but make it more complicated due to the fact that the plate is first placed on the orifices of the plate, in such a way that the place where the screws are inserted is determined by the geometry of the plate instead of being determined by the anatomical peculiarities of the individual. On the other hand, the plate conceals the vertebral body at the moment of inserting the screws, with the result that their place of insertion cannot be visually verified. Verification must be postponed until a radiographic inspection is made, once the screws have been inserted, with the risks this entails.
- The plate-screw set is subjected to a wide range of stresses due to the great mobility of the cervical rachis. These are mainly flexion-extension, but also torsion and lateral flexion, that result in the plates transmitting all the stresses on the screws inserted in their orifices, in such a way that these end up becoming detached from the osseous anchorage and are expelled from the vertebra, with the resulting danger for the nearby anatomical structures. Some systems solve this problem by increasing the length of the screws, which are also fastened on the posterior cortical. However, this technique increases even more the risk of the operation, as the back wall of the vertebra may be pierced and the screw may penetrate into the medullar channel. On the other hand, verification of the position of the screws in the back cortical can only be made by radiographic inspection.

### DESCRIPTION OF THE INVENTION

The aim of the invention constituting the purpose of this Patent consists in the elimination of the disadvantages of the known anterior cervical fastening systems described above, and has been conceived and designed with such purpose in mind.

To this effect the structure of said purpose has been designed to include the following elements as a minimum:
Four equal cervical screws inserted two-by-two on one and the other side of the anterior body of the two operated vertebras. The design of the head of the screws is such it permits these to be inserted before placing the plates. Furthermore, two different screw head designs will be made so that, once the closing element described later on is placed, free movement of the screw is permitted or not permitted with respect to the plate during movement caused by pure compression or by compression components associated to the above mentioned stresses.
Two equal and independent plates joining the screws of the same side of two or more operated cervical vertebras, provided with longitudinal grooves at their ends in such a way that the heads of the screws already inserted in the vertebras can be placed in different positions in said grooves and have a stiff transversal element that enables them to be joined one to another.
A transversal connecting element with a longitudinal groove placed on the plates once these are located on the heads of the screws, thus enabling the stresses to be transmitted from one plate to another.
Six closing nuts that have two functions: to be screwed on the head of the screw once the plate is placed in order to make the screw and plate in movement caused by flexion-extension, torsion and lateral flexion stresses integral and to make the transversal connecting element integral with the two plates by means of a rod screwed on to the center of each one, the projecting ends of which are fastened with nuts.

The described structure corresponds to the minimum efficiency and the number of screws, transversal elements and screwed closing elements may be increased according to the number of cervical vertebral levels to be operated on. In turn, the two sole plates will very in length and number of orifices as required by the chosen operation.

The way in which the "Anterior cervical fastening device" constituting the purpose of this Patent is able to overcome the disadvantages of the known devices is the following:
- If displacement of the screw as regards the plate is feasible during movement caused by pure compression or by compression components associated to the flexion-extension and lateral flexion stresses, the compression load passes to the graft that has to make the fusion, thus favoring it according to the Law of Wolff. For cases in which a certain stiffness is required during the erection (fractures or tumors), the surgeon may use one or more screws with the head design that does not permit displacement of the screws as regards the plate during movement caused by pure compression or by compression components associated to the flexion-extension and lateral flexion stresses.
- On being able to insert the screw into the vertebral body before placing the plate, this device permits the surgeon to decide the place of inserting the screws without it being concealed by the plate.
- As the device includes two independent plates, the surgeon can place the screws without having to bear in mind the geometry of the plate orifices, as only after placing the plates separately does the set become integral with the transversal connecting element.
- The closing nuts between the screw and plate allow the movements arising from the stresses not caused by compression to be restricted, thus avoiding removal of the screws from the vertebral body.

### BRIEF DESCRIPTION OF THE DRAWINGS

To complement the description of the invention and facilitate the interpretation of the formal, structural and functional characteristics of its purpose, attached are drawings in which different aspects of a preferred performance of the "Anterior cervical fastening device", constituting the purpose of this Patent, are schematically represented.

In said drawings:
Figure 1 shows a perspective view of the structure of the mounted device.
Figure 2 shows a perspective view of a cervical plate.
Figure 3 shows a section view of the distal and nearby parts of the cervical plate, cut by a plane the design of which is represented in Figure 2 as A-A.
Figure 4 shows a section view of the middle part of the cervical plate, cut by a plane the design of which is represented in Figure 2 as B-B.
Figure 5 shows a perspective view of the cervical screw with its head partially screwed and Figure 5' shows the head completely screwed for the stiffness of the device.

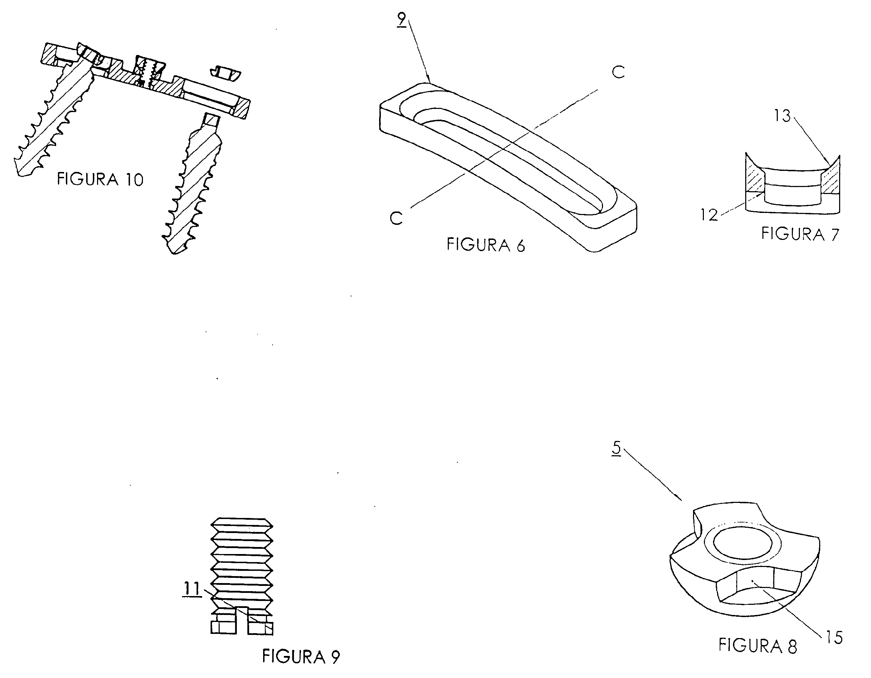

Figure 6 shows a perspective view of the transversal connection.
Figure 7 shows a section view of the transversal connection through the plane the design of which is represented as C-C in Figure 6.
Figure 8 shows a perspective view of the nut that screws on to the cervical screws and the central rod of the cervical plate.
Figure 9 shows an elevated view of the central rod of the cervical plate.
Figure 10 shows a section view of Figure 1, cut by the plane the design of which is represented as D-D.

### DESCRIPTION OF A PREFERRED PERFORMANCE

To clearly show the nature and scope of the advantageous application of the "Anterior cervical fastening device", purpose of the claimed invention, the following is a description of its structure and the characteristics of its component elements, making reference to the drawings which, on representing a preferred performance of said purpose for information, must be considered in the widest sense and not limiting of the application and content of the claimed invention.

The minimum efficient structure of the is composed of:
Four equal cervical screws (7) inserted two-by-two on one and the other side of the anterior body of the two operated vertebras.

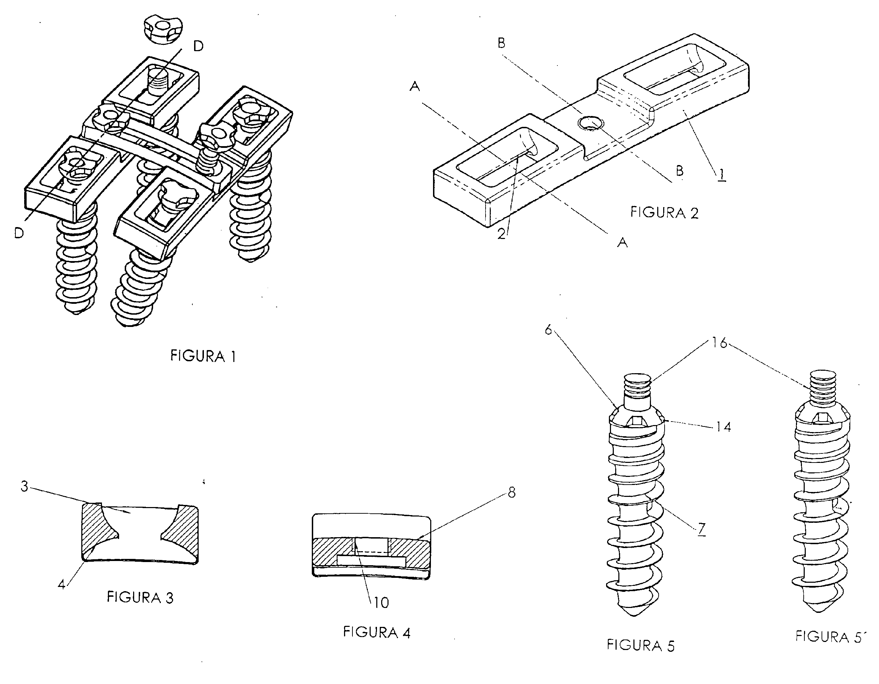

The head of said screws (7) is formed by a partially (Figure 5) or completely (Figure 5') screwed rod(16), in such a way that in the first case the nut (5) that screws on to said rod (16) will not tighten the plate (1) in the end grooves (2) of which it is inserted, thus permitting its movement in the direction of the compression stresses, restricting the movements in the other directions and preventing the forces exercised by the cervical rachis from causing the screws (7) to be expelled. In the second case, the nut (5) may apply pressure on the plate (1) and create a certain stiffness in the erection, convenient when dealing with fractures or tumors, thus preventing its movement in the direction of the compression stresses.

The head (6) of the screws (7), underneath the screwed rods (16), adopts the shape of a spherical segment (6) that adapts itself to the lower curvature (4) of the end grooves (2) of the plate (1). This segment (4) has notches (14) facilitating the grasping and screwing of the screws by means of forceps.

Two equal and independent plates (1) joining the cervical screws (7) of the same side of two or more operated cervical vertebras, provided with longitudinal grooves (2) at their ends in which the screwed heads (16) of the cervical screws (7) already inserted in the vertebras are introduced.

The transversal section of said grooves (2) shows a double inverse curvature (Figure 3): the upper (3) is concave, with the same radius as the curvature of the lower face of the nuts (5) where they are housed; and the lower (4) with the same radius as the spherical segment (6) of the head of the cervical screws (7).

The plates (1) also have central screwed orifices for inserting the screwed rods (11) that fasten the stiff transversal connection elements (9).

A stiff element (9) for transversal connection between the two plates (1), with a longitudinal groove (13) with a concave-curved section and radius equal to that of the lower face of the nuts (5).

Two screwed rods (11) that are inserted on to the center of each plate (1) from their lower face and the projecting ends of which are inserted into the longitudinal groove of the transversal element (9) and are fastened with special nuts (5).

Six special nuts (5) that are fastened on to the screwed head (15) of the four cervical screws (7) and on to the projecting ends of the two screwed rods (11).

The lower face of each nut (5) takes on the shape of a spherical segment that adapts itself to the upper curvature (3) of the end grooves (2) of the plates (1).

Said segment has notches (15) facilitating the grasping and screwing of the nuts by means of forceps.

## Claims

1. Anterior cervical fastening device, **characterized by** the fact that its minimum efficient structure is composed of four equal cervical screws (7); two equal plates (1); a stiff element (9) for transversal connection of the two plates (1); two screwed rods (11); and six special nuts (5). It has been provided that the four cervical screws (7) are inserted two-by-two on one and the other side of the anterior body of the two operated vertebras. The head of said screws (7) is formed by a partially (Figure 5) or completely (Figure 5') screwed rod, in such a way that in the first case the nut (5) that screws on to said rod (16) will not tighten the plate(1) in the end grooves (2) of which it is inserted, thus permitting its movement in the direction of the compression stresses, restricting the movements in the other directions and preventing the forces exercised by the cervical rachis from causing the screws (7) to be expelled. In the second case, the nut (5) may apply pressure on the plate (1) and create a certain stiffness in the erection, convenient when dealing with fractures or tumors, thus preventing its movement in the direction of the compression stresses.

2. Anterior cervical fastening device, according to Claim 1, **characterized by** the fact that the head (6) of the screws (7)), undemeath the screwed rods (16), adopts the shape of a spherical segment (6) that adapts itself to the lower curvature (4) of the end grooves (2) of the plate (1). This segment (4) has notches (14) facilitating the grasping and screwing of the screws by means of forceps.

3. Anterior cervical fastening device, according to Claim 1, **characterized by** the fact that the two equal and independent plates (1) joining the cervical screws (7) of the same side of two or more operated cervical vertebras are provided with longitudinal grooves (2) at their ends in which the screwed heads (16) of the cervical screws (7) already inserted in the vertebras are introduced. The transversal section of said grooves (2) shows a double inverse curvature (Figure 3): the upper (3) is concave, with the same radius as the curvature of the lower face of the nuts (5) where they are housed; and the lower (4) with the same radius as the spherical segment (6) of the head of the cervical screws (7). The plates (1) also have central screwed orifices for inserting the screwed rods (11) that fasten the stiff transversal connection elements (9).

4. Anterior cervical fastening device, according to Claim 1, **characterized by** the fact that the two screwed rods (11) are inserted on to the center of each plate (1) from their lower face and the projecting ends of which are inserted into the longitudinal groove of the transversal element (9) and are fastened with special nuts (5).

5. Anterior cervical fastening device, according to Claim 1, **characterized by** the fact that the two screwed rods (11) are inserted on to the center of each plate (1) from their lower face and the projecting ends of which are inserted into the longitudinal groove of the transversal element (9) and are fastened with special nuts (5).

6. Anterior cervical fastening device, according to Claim 1, **characterized by** the fact that the six special nuts (5) are fastened on to the screwed head (15) of the four cervical screws (7) and on to the projecting ends of the two screwed rods (11). The lower face of each nut (5) takes on the shape of a spherical segment that adapts itself to the upper curvature (3) of the end grooves (2) of the plates (1). Said segment has notches (15) facilitating the grasping and screwing of the nuts by means of forceps.
